# EUROPÄISCHE PATENTSCHRIFT

(11) **EP 3 592 390 B1**
(45) Veröffentlichungstag und Bekanntmachung des Hinweises auf die Patenterteilung: **14.05.2025**
(21) Anmeldenummer: 18714714.5
(22) Anmeldetag: 09.03.2018
(51) Int. Cl.: A61K 31/19, A61K 45/06, A61K 31/40, A61P 3/06

(54) **MITTEL ZUR ANWENDUNG BEI DER BEHANDLUNG VON DYSLIPIDÄMIE**
AGENT FOR USE IN THE TREATMENT OF DYSLIPIDEMIA
AGENT DESTINÉ À ÊTRE UTILISÉ POUR LE TRAITEMENT DE LA DYSLIPIDÉMIE

(30) Priorität: 09.03.2017 DE 102017105036
(43) Veröffentlichungstag der Anmeldung: 15.01.2020
(73) Patentinhaber: FLEXOPHARM BRAIN GmbH & Co. KG, 44628 Herne (DE)
(72) Erfinder: HAGHIKIA, Aiden, 44789 Bochum (DE)
(74) Vertreter: Schneiders & Behrendt Bochum
(86) Internationale Anmeldenummer: PCT/EP2018/055876
(87) Internationale Veröffentlichungsnummer: WO 2018/162698

(56) Entgegenhaltungen:
- JP-A- 2012 201 599
- TODESCO T ET AL: "Propionate lowers blood glucose and alters lipid metabolism in healthy subjects", AMERICAN JOURNAL OF CLINICAL NUTRITION,, vol. 54, 1 November 1991 (1991-11-01), pages 860 - 865, XP002402308, ISSN: 0002-9165
- HAGHIKIA ARASH ET AL: "Propionate attenuates atherosclerosis by immune-dependent regulation of intestinal cholesterol metabolism", EUROPEAN HEART JOURNAL, vol. 43, no. 6, 10 February 2022 (2022-02-10), GB, pages 518 - 533, ISSN: 0195-668X, DOI: 10.1093/eurheartj/ehab644
- P.A. THACKER ET AL: "EFFECTS OF VITAMIN B 12 ON SERUM LIPIDS AND LIPOPROTEINS OF PIGS FED DIETS SUPPLEMENTED WITH PROPIONIC ACID OR CALCIUM PROPIONATE", CANADIAN JOURNAL OF ANIMAL SCIENCE., vol. 62, no. 2, 1 June 1982 (1982-06-01), CA, pages 527 - 536, XP055481828, ISSN: 0008-3984, DOI: 10.4141/cjas82-062
- BERGGREN A M ET AL: "Influence of orally and rectally administered propionate on cholesterol and glucose metabolism in obese rats", BRITISH JOURNAL OF NUTRI, CAMBRIDGE UNIV. PRESS, UK, vol. 76, no. 2, 1 August 1996 (1996-08-01), pages 287 - 294, XP008100394, ISSN: 0007-1145
- DATABASE MEDLINE [online] US NATIONAL LIBRARY OF MEDICINE (NLM), BETHESDA, MD, US; May 1990 (1990-05-01), VENTER C S ET AL: "Effects of dietary propionate on carbohydrate and lipid metabolism in healthy volunteers.", XP002781888, Database accession no. NLM2159696
- THE AMERICAN JOURNAL OF GASTROENTEROLOGY MAY 1990, vol. 85, no. 5, May 1990 (1990-05-01), pages 549 - 553, ISSN: 0002-9270
- YUGUANG LIN ET AL: "Differences in propionate-induced inhibition of cholesterol and triacylglycerol synthesis between human and rat hepatocytes in primary culture", BRITISH JOURNAL OF NUTRITION, vol. 74, no. 02, 1 August 1995 (1995-08-01), UK, pages 197, XP055481847, ISSN: 0007-1145, DOI: 10.1079/BJN19950123
- DAVIDSON M.H. ET AL: "Efficacy and tolerability of atorvastatin/fenofibrate fixed-dose combination tablet compared with atorvastatin and fenofibrate monotherapies in patients with dyslipidemia: A 12-week, multicenter, double-blind, randomized, parallel-group study", CLINICAL THERAPEUTICS, EXCERPTA MEDICA, PRINCETON, NJ, US, vol. 31, no. 12, 1 December 2009 (2009-12-01), pages 2824 - 2838, XP026872216, ISSN: 0149-2918, [retrieved on 20100112], DOI: 10.1016/J.CLINTHERA.2009.12.007

## Beschreibung

Die Erfindung betrifft ein Mittel zur Verwendung bei der Behandlung überhöhter LDL-Werte.

Cholesterin ist ein lebenswichtiges Sterol, das im menschlichen Körper in einer Menge von bis zu 2 g pro Tag synthetisiert wird. Hinzu kommt mit der Nahrung aufgenommenes Cholesterin. Die körpereigene Cholesterinsynthese ist weitgehend entschlüsselt.

Physiologisch gesehen ist Cholesterin ein wichtiger Bestandteil der Plasmamembran. Es erhöht die Stabilität der Membran und trägt gemeinsam mit Proteinen dazu bei, Signalstoffe in die Zellmembran einzuschleusen und wieder hinaus zu befördern. Der menschliche Körper enthält etwa 140 g Cholesterin, wovon sich über 95 % innerhalb der Zellen und Zellmembranen befinden.

Cholesterin selbst ist fettlöslich und im Wasser unlöslich. Um die Zellen über das Blut versorgen zu können, wird es für den Transport an Lipoproteine gebunden. Diese können von unterschiedlicher Dichte sein und werden nach ihrem Verhalten beim Zentrifugieren unterteilt in Chylomikronen, VLDL, IDL, LDL, HDL und Lipoprotein a.

Cholesterin ist im Körper eine Vorstufe für Steroidhormone und Gallensäure. Neuere Forschungen zeigen zudem, dass der Körper Cholesterin zur Biosynthese herzwirksamer Glykoside nutzt.

Der Körper hält das Gleichgewicht zwischen benötigtem und vorhandenem Cholesterin über eine Reihe von Mechanismen aufrecht. Dies ist auf der einen Seite die Hemmung der HMG-CoA-Synthetase und -Reduktase, Enzymen der Cholesterin-Biosynthese, und auf der anderen Seite die Verstoffwechselung im Plasma, die Umwandlung in Gallensäure und teilweise Ausscheidung mit der Gallensäure und der Abbau im Plasma. Eine Störung dieses Gleichgewichts kann zu überhöhten Cholesterinwerten führen, die sich anhand des Cholesterinspiegels im Blut ermitteln lassen. Ein Gesamtcholesterinwert im Bereich von 190 bis 280 mg/dl gilt als normal.

Der Cholesterinstatus eines Menschen wird anhand des gemessenen Cholesterinwerts im Blut bestimmt. Nach den neuen Leitlinien zur Prävention für Herz-Kreislauf-Erkrankungen gilt, dass für gesunde Menschen mit einem Risiko das Gesamtcholesterin unter 190 mg/dl liegen sollte. Die empfohlenen LDL-Höchstwerte richten sich nach dem jeweiligen kardiovaskulären Risiko. Bei Patienten mit niedrigem bis mittlerem kardiovaskulären Risiko sollten die LDL-Werte 115 mg/dl nicht überschreiten, bei Patienten mit mittlerem bis hohem Risiko sollten die LDL-Werte 100 mg/dl nicht überschreiten und bei Patienten mit hohem kardiovaskulärem Risiko (z.B. bekannte koronare Herzerkrankung oder Diabetes mellitus) sollten die Werte 70 mg/dl nicht überschreiten, wobei der HDL-Wert mindestens 40 mg/dl betragen sollte. Hohe LDL-Werte gelten als Risikofaktor für Herzkreislauferkrankungen, hohe HDL-Werte gelten als Hinweis auf ein niedriges Risiko.

Der Gesamtcholesterinwert ermittelt in der Regel den Gesamtwert aller Cholesterinvarianten. Daneben wird in der Regel der HDL-Wert ermittelt und der LDL-Wert abgeschätzt anhand des Gesamtcholesterinwerts, vermindert um den Wert des HDL-Cholesterins und 20 % des Triglyceridwerts.

Zur Behandlung überhöhter Cholesterinwerte, insbesondere hoher Werte an LDL-Cholesterin wird auf einer Reihe von Mitteln zurückgegriffen. An erster Stelle zu nennen sind die Statine, die geeignet sind, in den Regulierungszyklus einzugreifen. Unter den Statinen hat sich in letzter Zeit das Atorvastatin weitgehend durchgesetzt, das in einer Standarddosierung in einer Menge von 10 bis 20 mg pro Tag verabreicht wird, in Fällen schwerer Hypercholesterinämie in einer Menge von bis zu 80 mg/Tag. Atorvastatin hat allerdings, wie andere Statine auch, eine Reihe von Nebenwirkungen, beispielsweise Störungen des Magen-Darmtrakts, Müdigkeit, Muskelschmerzen [Statin-assoziierte Muskelsymptome (SAMS)], Anstieg der Leberenzyme sowie Kopf- und Gelenkschmerzen. Hinzu kommen selten toxische Myopathien und ein erhöhtes Diabetesrisiko, wobei die Risiken von der FDA als schwerwiegend eingestuft werden. Ein Problem ist dabei, dass die Risiken mit der Dosierung einhergehen und bei der Normaldosis bereits als erheblich eingestuft werden.

Die Cholesterinwerte eines Menschen lassen sich zudem durch die Ernährung beeinflussen, insbesondere durch faserreiche Kost und Hefeprodukte. Diese an und für sich wirksame Beeinflussung reicht aber bei stark erhöhten Cholesterinwerten zumeist nicht aus.

Bei der Verstoffwechselung von Pflanzenfasern entsteht in geringen Mengen Propionsäure. Dies hat dazu geführt, Propionsäure und ihre Derivate auf eine Wirksamkeit bei Hypercholesterinämie hin zu untersuchen. In einer Reihe von Studien wurden widersprüchliche Ergebnisse erzielt.

Von Chen et al, Proceedings of the Society for Experimental Biology and Medicine, 175, 215 - 218 (1984) wurde der Effekt der Verabreichung von Natriumpropionat auf den Cholesterinwert von Ratten bei Normalkost und cholesterinreicher Kost untersucht. Wurde das Propionat bei cholesterinreicher Kost verabreicht, ergab sich eine signifikante Senkung des Gesamtcholesterins, während bei normal ernährten Ratten die Zugabe von Propionat praktisch keinen Effekt zeigte. In der Studie wurde der LDL-Wert nicht bestimmt. Die verabreichte Dosis an Natriumpropionat betrug etwa 100 mg pro Tag, was, bezogen auf den Menschen, einer Tagesdosis von etwa 30 g entspricht.

Den Autoren ist die Ambivalenz ihrer Ergebnisse bewusst; sie verweisen auf eine Studie, in der Schweine mit vergleichsweise höheren Dosen an Propionat gefüttert wurden und, bei vermindertem Gesamtcholesterin, eine leichte Erhöhung des Cholesterinanteils in der Leber und eine signifikant höhere Cholesterinkonzentration im Rückenfett nachwiesen.

Berggren et al, British Journal of Nutrition (1996), 76, 287 - 294, zeigten, dass die orale und rektale Verabreichung von hohen Mengen Natriumpropionat an Ratten den Cholesteringehalt der Leber nur unwesentlich beeinflussen. In der Studie wird auf humane Studien verwiesen, wonach in einem Fall die Verabreichung von Propionat an Patienten in hohen Dosen eine geringfügige Verringerung des Serum-Cholesterinwerts bewirkte, in einem zweiten Fall die HDL-Serum-Cholesterinkonzentration erhöhte.

Lin et al, British Journal of Nutrition (1995), 74, 197 - 207, verweisen auf große Unterschiede bei der Inhibierung der Cholesterin- und Triacylglycerinsynthese in Hepatozyten bei Mensch und Ratte.

Aus der JP 2012 201599 A ist die Nützlichkeit Propionsäure-produzierender Bakterien zur Behandlung von erhöhten LDL-Werten im Menschen bekannt. Die Bakterien werden im abgetöteten Zustand verabreicht.

T.Todesco et al, Propionate lowers blood glucose and alters lipid metaboslism in healthy subjects, Am. J. of Clin. Nutr., Bd 54, 860-865, befasst sich mit der physiologischen Wirkung von mit Propionat konserviertem Brot.

Insgesamt ergibt sich, dass die Verabreichung von Propionat einen Effekt auf den Cholesterinstoffwechsel eines Lebewesens ausübt, dieser Effekt bislang aber nur bedingt quantifiziert wurde.

Es wurde jetzt gefunden, dass eine kontrollierte Verabreichung von Propionat durchaus geeignet ist, in die Cholesterinsynthese und den Abbau von Cholesterin in einem Körper steuernd einzugreifen. Dabei ergaben sich positive Effekte vor allem in der Kombination mit Statinen.

Grundsätzlich wäre es wünschenswert, über ein alternatives Mittel zu verfügen, mit dem die cholesterinsenkende Wirkung von Statinen erreicht werden kann oder das zusammen mit Statinen dazu führt, dass die Statindosis nennenswert gesenkt werden kann.

Es wurde gefunden, dass Propionsäure oder deren physiologisch vertretbare Salze eine prophylaktische oder therapeutische Wirkung auf hohe Cholesterinwerte ausüben. Insbesondere sind sie geeignet, den LDL-Serumspiegel auf gesundheitlich vertretbare Größen zu senken.

Entsprechend betrifft die Erfindung ein Mittel zur Verwendung bei der Behandlung überhöhter LDL-Werte im Menschen, enthaltend Propionsäure oder ein physiologisch vertretbares Propionsäuresalz in einer Tagesdosis von 0,5 bis 1,5g.

Propionsäure, CH₃-CH₂-COOH, ist eine kurzkettige Fettsäure, die in geringen Mengen von der Darmflora gebildet wird und bei der Verdauung von faserreicher Rohkost generiert wird. Sie dient in der Nahrungsmittelindustrie als Konservierungsmittel, vor allem für Teigwaren, und wurde früher zu Konservierung von Brot in großem Umfang eingesetzt. Inzwischen ist durch moderne Fertigungs- und Verpackungsmethoden die Zugabe von Konservierungsmitteln zu Brot weitgehend entfallen.

Unter Propionsäuresalzen werden deren physiologisch vertretbare Salze vertreten, beispielsweise die Alkali- und Erdalkalisalze und insbesondere das Natrium-, Kalium-, Calcium- und Magnesiumsalz. Weiterhin können Salze anderer essentieller Metalle verwandt werden, beispielsweise von Eisen oder Zink, wie auch das Ammoniumsalz und Salze von organischen Aminen. Schließlich kommen auch beliebige Mischungen dieser Salze in Frage.

Die Propionsäure oder ihr Salz wird in einer Tagesdosis von 0,5 g bis 1,5 gverabreicht, beispielsweise in Form von zwei Verabreichungen von 0,5 g morgens und abends. Eine Einheitsdosis enthält 200 bis 1.500 mg Wirkstoff, insbesondere 250 bis 1.000 mg.

Als Verabreichungsform kommen in der Regel Kapseln oder Tabletten in Frage. Eine Verabreichung in Pulverform, enthalten beispielsweise in Sachets, ist ebenfalls möglich. Eine Kapsel, Tablette oder ein Sachet kann 0,2 bis 1,0 g Wirkstoff, beispielsweise 0,5 g Propionsäuresalz enthalten.

Es ist festzuhalten, dass die Propionsäurederivate nach der Verabreichung im Körper überwiegend in die freie Propionsäure überführt werden, die im Darm ihre Wirkung entfaltet.

Kapseln, Tabletten und Sachets können den Wirkstoff zusammen mit üblichen Träger- und Hilfsstoffen enthalten.

Besonders bevorzugt ist die Kombination der Propionsäure oder ihres Salzes mit einem Statin. Diese Kombination erlaubt es, die Statindosis auf die Hälfte der üblichen Tagesdosis oder weniger zu senken. Für die Kombination kommen alle Statine in Frage, insbesondere aber Atorvastatin.

Für die Dosierung in Kombination mit Atorvastatin liegt die Dosierung beispielsweise bei 2 x 0,5 g Propionsäuresalz und 5 bis 50 mg, vorzugsweise 5 bis 20 mg und insbesondere 5 bis 10 mg Atorvastatin. Üblicherweise wird Atorvastatin in Dosen von 10 bis 20 mg oder mehr verabreicht, je nach Schwere der Hypercholesterinämie, bis zu 80 mg täglich. Vorzugsweise wird dabei das Propionsäurederivat morgens und abends verabreicht, das Atorvastatin abends, ebenfalls wie üblich.

Für die Verabreichung der Kombination ist es sinnvoll, die Wirkstoffe separat in Blisterpackungen anzubieten, wobei eine Tagesdosis aus zwei Kapseln oder Tabletten Propionsäuresalz und einer Tablette Atorvastatin besteht.

Die Erfindung wird durch die angehängten Darstellungen näher erläutert. Es zeigen:

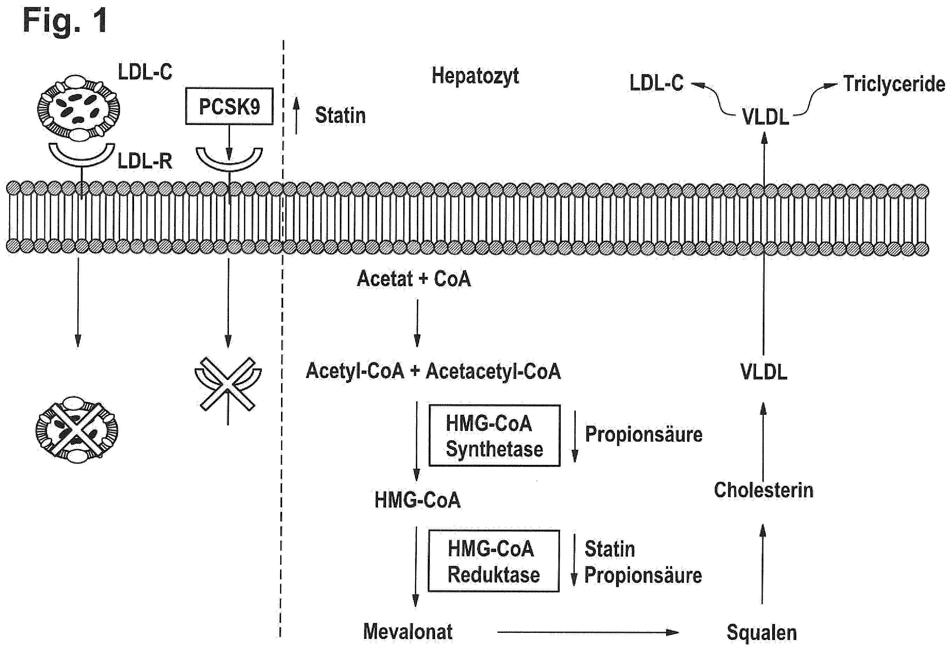

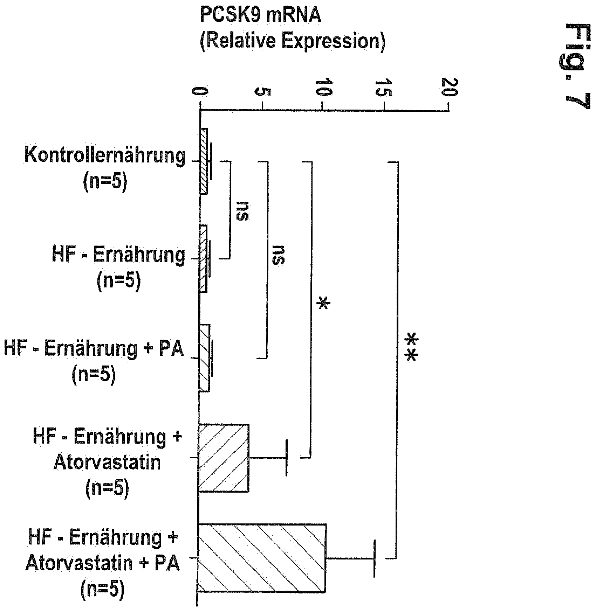

- Fig. 1: zeigt ein Diagramm zur Cholesterinsynthese/-abbau in Hepatozyten. Die Aufnahme des LDL-Cholesterins (LDL-C) in die Hepatozyten mit konsekutivem Abbau erfolgt über LDL-Rezeptoren (LDL-R) an der Zelloberfläche. Die Degradation der LDL-Rezeptoren wiederum wird u.a. durch die Protease Proproteinkonvertase Subtilisin/Kexin Typ 9 (PCSK9) gefördert. Während die Expression des PCSK9 bekannterweise durch Statine erhöht wird, hat die Propionsäure in unseren Experimenten keinen Einfluss auf die PCSK9 Expression (s. Fig. 7). Wir zeigen, dass die Expression von zwei Schlüsselenzymen der Cholesterinsynthese durch die Propionsäure negativ reguliert werden: a) 3-Hydroxy-3-Methylglutaryl-Coenzym-A-Synthase (HMG-CoA-Synthase) und b) HMG-CoA-Reduktase.
- Fig. 2: zeigt die Reduktion des LDL-Cholesterins durch Propionat im hypercholesterinämischem Mausmodell. HF: High Fat (60 KJ% Fettanteil); PA: Propionic acid.

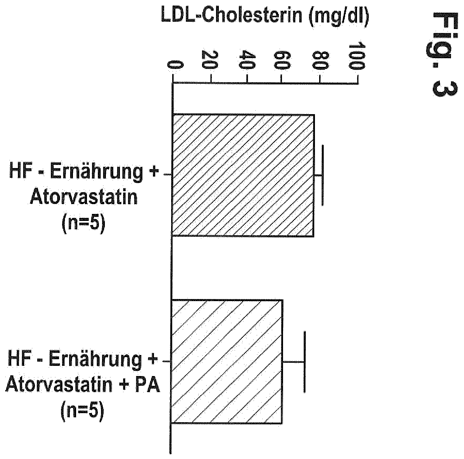

- Fig. 3: zeigt einen Trend zur weiteren Reduktion des LDL-Cholesterins durch Propionsäure bei zusätzlicher Gabe zum Atorvastatin; HFD: High Fat Diet; PA: Propionic acid.

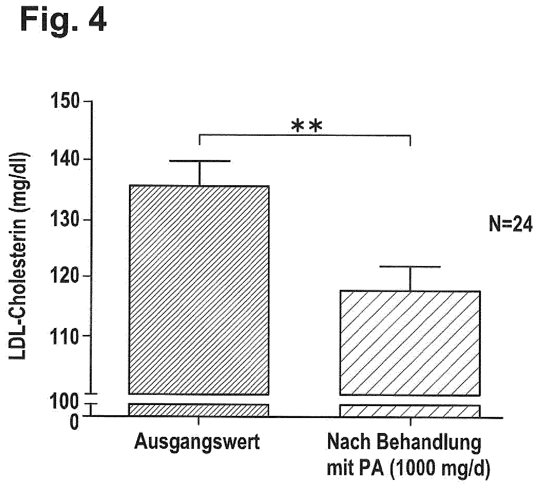

- Fig. 4.: zeigt die Cholesterin-senkende Wirkung der Propionsäure bei dyslipidämischen Patienten (n=24, davon 11 männlich u. 13 weiblich; Alter 21-70 J) mit einem initialen LDL-Cholesterinwert von > 110mg/dl nach 2-6 Monaten Behandlung (n=24).
- Fig. 5: zeigt eine reduzierte Expression mittels Reverse Transkriptase-Polymerase-Kettenreaktion der HMG-CoA-Synthase auf mRNA Ebense durch die Propionsäure aber nicht durch das Atorvastatin.
- Fig. 6: zeigt eine reduzierte Expression der HMG-CoA-Reduktase durch die Propionsäure mittels Reverse Transkriptase-Polymerase-Kettenreaktion. Dieser Effekt wird ebenfalls durch das Atorvastatin beobachtet.
- Fig. 7: zeigt eine erhöhte Expression des PCSK9 mittels Reverse Transkriptase-Polymerase-Kettenreaktion durch das Atorvastatin. Die Propionsäure hat jedoch keinen Einfluss auf die PCSK9 Expression.

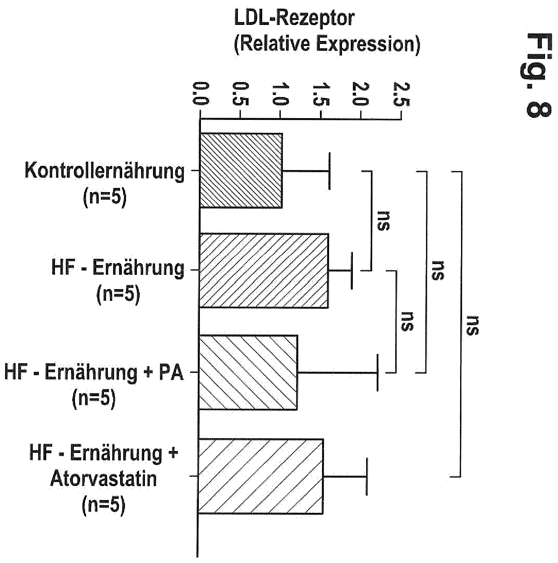

- Fig. 8: zeigt die Expression des LDL-Rezeptors mittels Reverse Transkriptase-Polymerase-Kettenreaktion in der Leber nach Behandlung mit Propionsäure oder Atorvastatin. Weder die Propionsäure noch das Atorvastatin beeinflussen die Expression des LDL-Rezeptors.

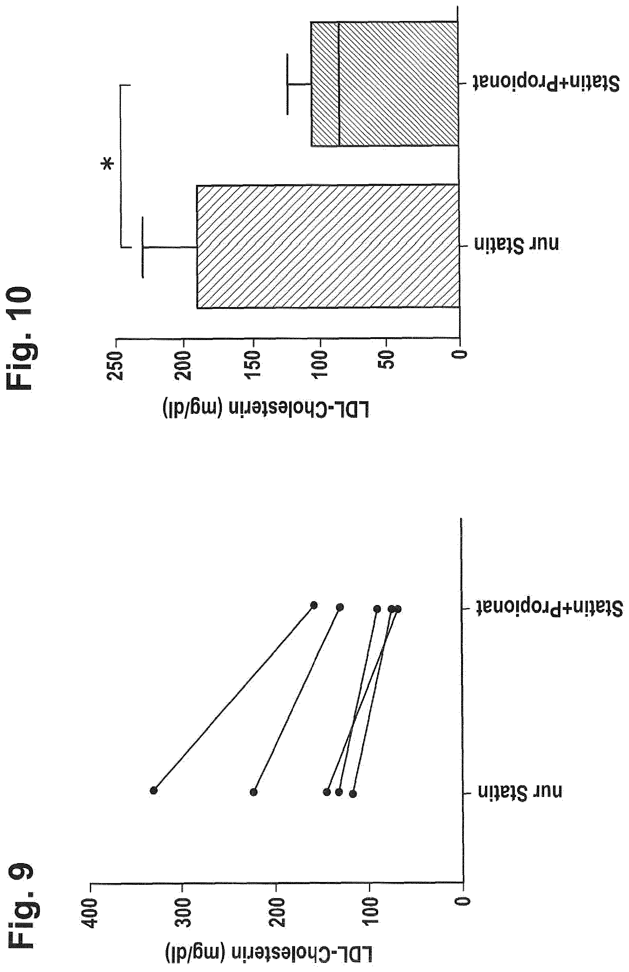

- Fig. 9: zeigt die Auswertung einer Studie an fünf Patienten, deren LDL-Cholesterinwert mit Atorvastatin auf ein stabiles Niveau eingestellt worden war. Nach mehr als achtwöchiger Einnahme von 2 x 500 mg Propionat ergab sich die im Diagramm angezeigte Reduktion des LDL-Wertes. Die Abnahme scheint bei Patienten mit dem höchsten LDL-Ausgangswert am größten zu sein.
- Fig. 10: zeigt das Ergebnis einer Patientenstudie als Blockdiagramm. Links ist der gemittelte Ausgangswert der mit Atorvastatin stabil eingestellten Patienten dargestellt, rechts der gemittelte LDL-Wert der Patienten nach mindestens achtwöchiger Einnahme von 2 x 500 mg Propionat täglich. Es ergibt sich im Mittel eine Reduktion des LDL-Wertes um nahezu 40 %.

Die Proproteinkonvertase PCSK9 wird von Propionaten nicht beeinflusst. PCSK9 besitzt klinische Bedeutung, da es die Anzahl von LDL-Rezeptoren an der Zellmembran der Leberzellen mindert. Über LDL-Rezeptoren wird das LDL-Cholesterin in die Leber aufgenommen und abgebaut. (Fig. 1) Eine erhöhte Expression des PCSK9 führt folglich zur erhöhten Konzentrationen von LDL-Cholesterin im Blut. Einer der entscheidenden Nachteile von Atorvastatin ist die Steigerung der PCSK9-Expression, was die Cholesterin-senkende Wirkung begrenzt. Dieser negative Effekt ist bei Propionaten nicht nachzuweisen.

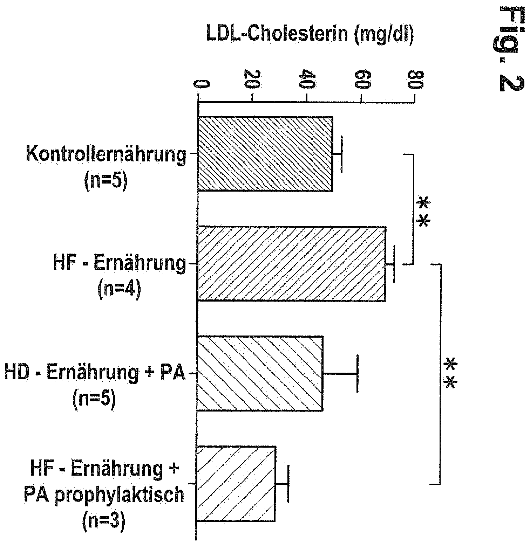

Fig. 7 zeigt, dass die Kombination von Propionat mit Atorvastatin zu einer noch weiteren Steigerung der PCSK9-Expression führt, verglichen mit Atorvastatin alleine. Allerdings führt dies nicht zu einer Steigerung der LDL-Cholesterinkonzentration im Blut, wie zu erwarten wäre, sondern zu einer Absenkung, siehe Fig. 2.

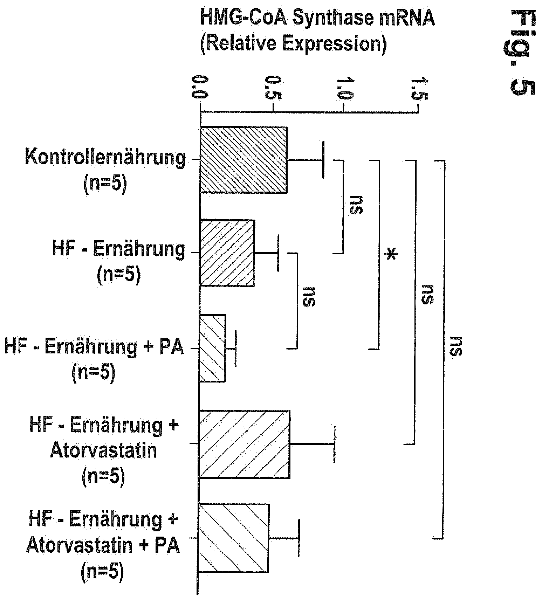

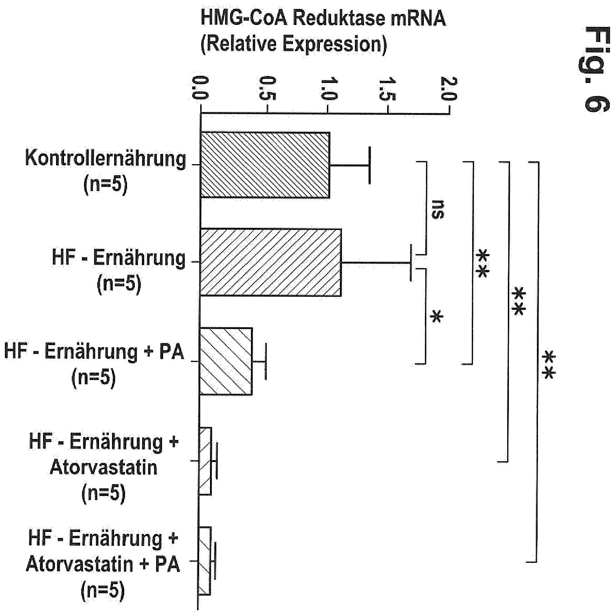

Fig. 5 und 6 zeigen einen massiven Einfluss von Propionaten auf die Expression von HMG-Co-A-Synthase mRNA und Reduktase mRNA. Beide Enzyme werden durch Propionat deutlich herunter reguliert, was sich dämpfend auf die Cholesterinsynthese auswirkt. Bei der Synthase ist die Auswirkung von Propionat deutlich ausgeprägter als bei Atorvastatin, bei der Reduktase hat Atorvastatin den größeren Einfluss.

Die Patientenstudie zeigt den positiven Einfluss von 2 x 500 mg Natriumpropionat zusätzlich zu Atorvastatin. Die LDL-Cholesterinwerte können über das mit regelmäßiger Verabreichung von Atorvastatin erreichte stabile Niveau weiter erheblich abgesenkt werden, wobei die Abnahme bei hohen LDL-Werten am größten zu sein scheint.

## Patentansprüche

1. Mittel zur Verwendung bei der Behandlung überhöhter LDL-Werte im Menschen, enthaltend Propionsäure oder ein physiologisch vertretbares Propionsäuresalz in einer Tagesdosis von 0,5 bis 1,5g.

2. Mittel zur Verwendung bei der Behandlung überhöhter LDL-Werte im Menschen nach Anspruch 1, **dadurch gekennzeichnet, dass** das Propionsäurederivat ein Alkali- oder Erdalkalisalz der Propionsäure ist.

3. Mittel zur Verwendung bei der Behandlung überhöhter LDL-Werte im Menschen nach Anspruch 2, **dadurch gekennzeichnet, dass** das Propionsäuresalz Natrium-, Kalzium- oder Magnesiumpropionat ist.

4. Mittel zur Verwendung bei der Behandlung überhöhter LDL-Werte im Menschen nach einem der vorstehenden Ansprüche in Form einer Tablette, einer Kapsel oder eines Sachets.

5. Mittel zur Verwendung bei der Behandlung überhöhter LDL-Werte im Menschen nach einem der vorstehenden Ansprüche in Form einer Einheitsdosis von 200 bis 1.500 mg Wirkstoff.

6. Mittel nach Anspruch 5 mit einer Einheitsdosis von 250 bis 1.000 mg.

7. Mittel zur Verwendung bei der Behandlung überhöhter LDL-Werte im Menschen nach einem der vorstehenden Ansprüche, **dadurch gekennzeichnet, dass** es einen Trägerstoff enthält.

8. Mittel zur Verwendung bei der Behandlung überhöhter LDL-Werte im Menschen nach einem der vorstehenden Ansprüche in Kombination mit einem Statin.

9. Mittel zur Verwendung bei der Behandlung überhöhter LDL-Werte im Menschen nach Anspruch 8, **dadurch gekennzeichnet, dass** das Statin Atorvastatin ist.

10. Mittel zur Verwendung bei der Behandlung überhöhter LDL-Werte im Menschen nach Anspruch 12, **dadurch gekennzeichnet, dass** es 5 bis 50 mg, vorzugsweise 5 bis 20 mg und insbesondere 5 bis 10 mg, Atorvastatin enthält.

11. Mittel zur Verwendung bei der Behandlung überhöhter LDL-Werte im Menschen nach einem der Ansprüche 9 bis 12, **dadurch gekennzeichnet, dass** es ein Propionsäuresalz und ein Statin getrennt voneinander in einer Blisterpackung enthält.

12. Mittel zur Verwendung bei der Behandlung überhöhter LDL-Werte im Menschen nach Anspruch 11, **dadurch gekennzeichnet, dass** die Blisterpackung 2 x 0,5 g Calcium- oder Magnesiumpropionat und 5 mg Atorvastatin enthält.

## Claims

1. Agent for use in the treatment of excessive LDL levels in humans, containing propionic acid or a physiologically acceptable propionic acid salt in a daily dosage of 0.5 to 1.5 g.

2. Agent for use in the treatment of excessive LDL levels in humans according to claim 1, **characterized in that** the propionic acid derivative is an alkali or alkaline earth salt of the propionic acid.

3. Agent for use in the treatment of excessive LDL levels in humans according to claim 2, **characterized in that** the propionic acid salt is sodium, calcium or magnesium propionate.

4. Agent for use in the treatment of excessive LDL levels in humans according to any one of the preceding claims in the form of a tablet, a capsule or a sachet.

5. Agent for use in the treatment of excessive LDL levels in humans according to any one of the preceding claims in the form of a unitdose of between 200 and 1500 mg of active substance.

6. Agent according to claim 5 with a unitdose of between 250 and 1000 mg.

7. Agent for use in the treatment of excessive LDL levels in humans according to any one of the preceding claims, **characterized in that** it contains a carrier substance.

8. Agent for use in the treatment of excessive LDL levels in humans according to any one of the preceding claims in combination with a statin.

9. Agent for use in the treatment of excessive LDL levels in humans according to claim 8, **characterized in that** the statin is atorvastatin.

10. Agent for use in the treatment of excessive LDL levels in humans according to claim 12, **characterized in that** it contains 5 to 50 mg, preferably 5 to 20 mg and in particular 5 to 10 mg of atorvastatin.

11. Agent for use in the treatment of excessive LDL levels in humans according to any one of claims 9 to 12, **characterized in that** it contains a propionic acid salt and a statin kept separately from each other in a blister pack.

12. Agent for use in the treatment of excessive LDL levels in humans according to claim 11, **characterized in that** the blister pack contains 2 x 0.5 g of calcium or magnesium propionate and 5 mg of atorvastatin.

## Revendications

1. Agent destiné à être utilisé pour le traitement des valeurs LDL excessives chez l'homme, contenant de l'acide propionique ou un sel d'acide propionique physiologiquement acceptable à une dose journalière de 0,5 à 1,5 g.

2. Agent destiné à être utilisé dans le traitement des valeurs LDL excessives chez l'homme selon la revendication 1, **caractérisé en ce que** le dérivé de l'acide propionique est un sel alcalin ou alcalino-terreux de l'acide propionique.

3. Agent destiné à être utilisé dans le traitement des valeurs LDL excessives chez l'homme selon la revendication 2, **caractérisé en ce que** le sel d'acide propionique est du propionate de sodium, de calcium ou de magnésium.

4. Agent destiné à être utilisé dans le traitement des valeurs LDL excessives chez l'homme selon l'une des revendications précédentes sous forme de comprimé, de capsule ou de sachet.

5. Agent destiné à être utilisé dans le traitement des valeurs LDL excessives chez l'homme selon l'une des revendications précédentes, sous la forme d'une dose unitaire de 200 à 1 500 mg de principe actif.

6. Agent selon la revendication 5 avec une dose unitaire de 250 à 1000 mg.

7. Agent destiné à être utilisé dans le traitement des valeurs LDL excessives chez l'homme selon l'une des revendications précédentes, **caractérisé en ce qu'**il contient un excipient.

8. Agent destiné à être utilisé dans le traitement des valeurs LDL excessives chez l'homme selon l'une des revendications précédentes en combinaison avec une statine.

9. Agent destiné à être utilisé dans le traitement des valeurs LDL excessives chez l'homme selon la revendication 8, **caractérisé en ce que** la statine est l'atorvastatine.

10. Agent destiné à être utilisé dans le traitement des valeurs LDL excessives chez l'homme selon la revendication 12, **caractérisé en ce qu'**il contient 5 à 50 mg, de préférence 5 à 20 mg et en particulier 5 à 10 mg d'atorvastatine.

11. Agent destiné à être utilisé dans le traitement des valeurs LDL excessives chez l'homme selon l'une des revendications 9 à 12, **caractérisé en ce qu'**il contient un sel d'acide propionique et une statine séparés l'un de l'autre dans un emballage blister.

12. Agent destiné à être utilisé dans le traitement des valeurs LDL excessives chez l'homme selon la revendication 11, **caractérisé en ce que** l'emballage blister contient 2 x 0,5 g de propionate de calcium ou de magnésium et 5 mg d'atorvastatine.
